# EUROPEAN PATENT APPLICATION

(11) **EP 1 020 449 A1**
(43) Date of publication of application: **19.07.2000**
(21) Application number: 98943007.9
(22) Date of filing: 16.09.1998
(51) Int. Cl.: C07D 233/06, A61K 31/415

(54) **IMIDAZOLINE COMPOUNDS**

(30) Priority: 18.09.1997 JP 25356897
(71) Applicant: Mitsubishi-Tokyo Pharmaceuticals, Inc., Chuo-ku, Tokyo 103-8405 (JP)
(72) Inventor: OHNO, Norio Tokyo Tanabe Company Limited, Tokyo 103-8405 (JP); MIURA, Masataka Tokyo Tanabe Company Limited, Chuo-ku Tokyo 103-8405 (JP); AIZAWA, Hideyuki Tokyo Tanabe Company Limited, Chuo-ku Tokyo 103-8405 (JP)
(74) Representative: Bühling, Gerhard, Dipl.-Chem.
(86) International application number: JP9804162
(87) International publication number: WO9914200

(57) **Abstract**

The imidazoline compound represented by the following formula (I): 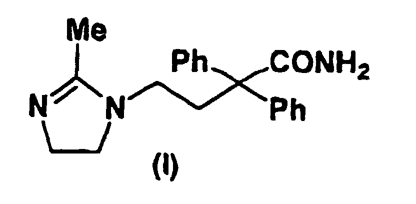 and its pharmacologically acceptable salts, and the imidazoline compound represented by the following formula (II):

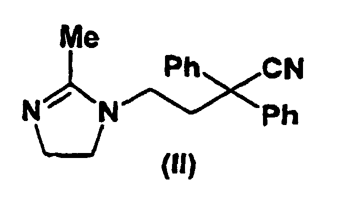

Compound (I) has highly selective antagonistic action on muscarinic M₃ receptor subtypes, and especially airway muscarinic M₃ receptor subtypes, and is, therefore, particularly useful as a preventive or therapeutic agent for especially, diseases related to muscarinic M₃ receptors in chronic obstructive pulmonary disease and asthma, while compound (II) is useful as a production intermediate for compound (I).

## Description

### Technical Field

The present invention relates to imidazoline compounds. Specifically, it relates to an imidazoline compound with selective antagonistic action on muscarinic M₃ receptor subtypes and particularly with muscarinic M₃ antagonistic action that is highly specific, its pharmacologically acceptable salts, pharmaceutical compositions containing it as an effective component, and to a novel imidazoline compound (II) that is useful as a production intermediate for compound (I).

Compound (I) is especially useful as a preventive or therapeutic agent for diseases related to muscarinic M₃ receptors, particularly chronic obstructive pulmonary disease (hereunder, "COPD") and asthma.

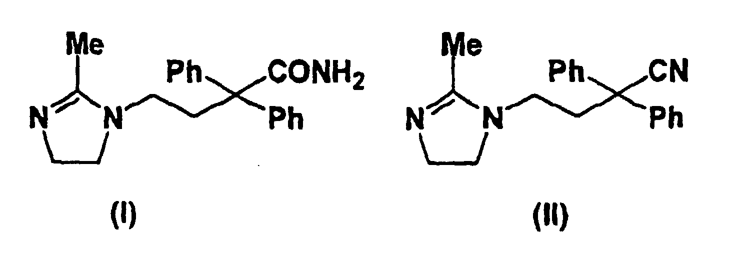

### Background Art

Muscarinic receptors exist as at least three different subtypes, of which it has been reported that muscarinic M₁ receptor subtypes are predominantly distributed in the brain, muscarinic M₂ receptor subtypes are distributed in the heart and muscarinic M₃ receptor subtypes are distributed in smooth muscle and glandular tissue.

It has been demonstrated that muscarinic M₁, M₂ and M₃, receptor subtypes are distributed among airway muscarinic receptors (Trends in Pharmacological Sciences, 1988, 9, 412), and it is known that M₁ receptor subtypes are present in the sympathetic ganglia and parasympathetic ganglia, M₂ receptor subtypes are present in pulmonary cholinergic nerve terminals, and there are play a role in acetylcholine release regulating (presynaptic inhibiting receptors) and M₃, receptor subtypes are present in smooth muscle (postsynaptic receptors).

COPD is a disease classification characterized by symptoms of breathing difficulties, with the main clinical feature being physiological obstructive ventilatory impairment, and includes such conditions as pulmonary emphysema, chronic bronchitis and peripheral airway disease.

Current treatment of COPD involves the use of the anti muscarinic bronchodilators ipratropium, flutropium, oxytropium and the like. These drugs, however, compete with acetylcholine non-selectively for muscarinic receptor subtypes in the airway. This means those there effects as bronchodilators are attenuated, while it is impossible to avoid side effects due to antagonism on other receptor subtypes than muscarinic M₃ receptor subtypes.

It has therefore been desirable to provide bronchodilators with highly receptor-selective antagonistic action on muscarinic receptors, and especially drugs that do not have attenuated efficacy due to muscarinic M₂ receptor antagonism or induce heart-related side-effects due to muscarinic M₂ receptor antagonism.

Japanese Laid Open Patent Publication No. 1-128970 describes the following heterocyclic derivatives with anti-muscarinic action 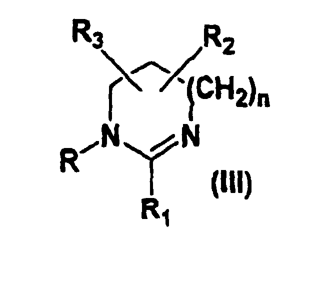 where R is a hydrogen atom or C₁₋₄ alkyl optionally substituted with 2 or 3 of the same or different groups selected from among aryl, cycloalkyl, hydroxyl and carboxamide;
R₁ is R or NHR₄ [where R₄ is a hydrogen atom, an -OOCR₅ group (in which R₃ is methyl optionally substituted with 2 or 3 of the same or different groups selected from among aryl, cycloalkyl and hydroxyl) or a cycloalkyl group substituted with a different cycloalkyl group];
R₂ is a hydrogen atom, C₁₋₄ alkyl or -OOCR₅ group (where R₅ is as defined above);
R₃ is a hydrogen atom or C₁₋₄ alkyl, and n is 0, 1 or 2; provided that at least one among R, R₁, R₂ and R₃ is not a hydrogen atom.

However, this publication provides no concrete disclosure where R is C₁₋₄ alkyl substituted with aryl and carboxamide (hereunder referred to as carbamoyl). Specifically, it does not concretely disclose the feature of compound (I) whereby R is propyl substituted with phenyl and carbamoyl, i.e. 3-carbamoyl-3,3-diphenylpropyl. Furthermore, while it mentions antagonistic action on muscarinic receptors in the cerebral cortex and ileum, i.e. muscarinic M₁ and M₃ receptor subtypes, it nowhere mentions antagonistic action on muscarinic M₂ receptor subtypes. In addition, while it discusses the usefulness as a therapeutic agent for gastrointestinal motility disturbance and urinary spasms, it nowhere suggests usefulness for COPD or asthma due to muscarinic M₃ receptor subtypes antagonism, which is the object of the present invention.

In Farmaco (1991, 46(4), 539-53), the selectivity in the antagonistic action of compound (III) of the aforementioned publication on muscarinic M₁, M₂ and M₃ receptor subtypes are described. For example, compound (IV) 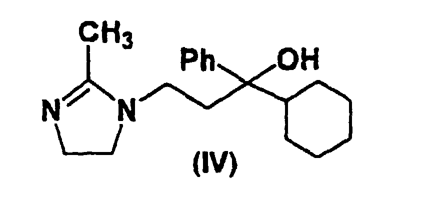 exhibits its most powerful antagonistic action on muscarinic M₁ receptor subtypes, followed by muscarinic M₃ and M₂ receptor subtypes. Specifically, if the antagonistic action on muscarinic M₁ receptor subtypes is defined as 1, then muscarinic M₃ receptor subtypes are 0.5 and muscarinic M₂ receptor subtypes is 0.06. Consequently, compound (IV) has selective antagonistic action on muscarinic M₁ receptor subtypes over muscarinic M₃ receptor subtypes. Also, the muscarinic M₃ receptor subtypes described in the article are found in the submaxillary gland and ileum, and there is absolutely no mention of airway muscarinic M₃ receptor subtypes.

On the other hand, Japanese Laid Open Patent Publication No. 7-215943 discloses imidazole derivatives with high selectivity and powerful antagonistic action on smooth muscle muscarinic receptors over cardiac muscarinic receptors, and more specifically, compounds of formula (V) below wherein an imidazole ring and substituted methyl are bonded via a C₁₋₆ alkylene group 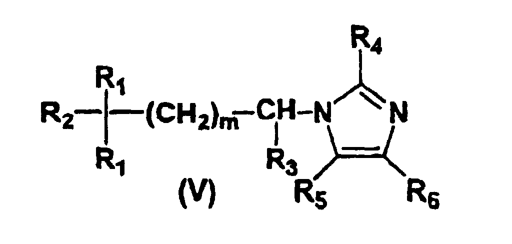 where each R₁ represents an optionally substituted phenyl or thienyl group, R₂ represents a cyano, hydroxyl, carboxyl, CONR₇R₈ (where R₇ and R₈ may be the same or different and each represent a hydrogen atom or lower alkyl group, or R₇ and R₈ are alkylene chains that form a ring optionally containing a hetero atom) or COOR₉ group (where R₉ represents a lower alkyl group), R₃ represents a hydrogen atom or a lower alkyl group, R₄, R₅ and R₆ may be the same or different and each represents a hydrogen atom or an optionally substituted lower alkyl or cycloalkyl group, with optional ring fusion with a benzene ring at the R₅ and R₆ positions, and m represents an integer of 1 to 6. However, the structure of compound (I) is clearly different since it has an imidazoline ring and carbamoyldiphenylmethyl bonded via an ethylene group. Furthermore, as will be described hereunder, compound (VI) 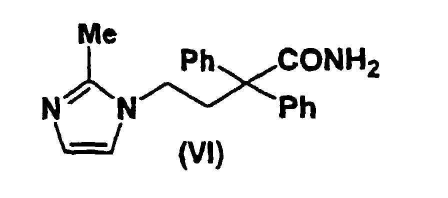 described in Example 11 of the aforementioned publication exhibits virtually no tissue specificity in muscarinic M₃ receptor subtypes antagonism, whereas compound (I) has powerful antagonistic action on airway muscarinic M₃ receptor subtypes (see Table 1).

It is therefore an object of the present invention to provide an imidazoline compound with receptor selectivity for muscarinic M3 receptor subtypes and airway tissue specificity, as well as its pharmacologically acceptable salts, a pharmaceutical composition for prevention or therapy of COPD or asthma, and a novel imidazoline compound (II) as an intermediate suitable for the production of compound (I).

### Disclosure of the Invention

As a result of much diligent research aimed at achieving the object stated above, the present inventors have completed the present invention upon the discovery of a novel imidazoline compound (I) represented by 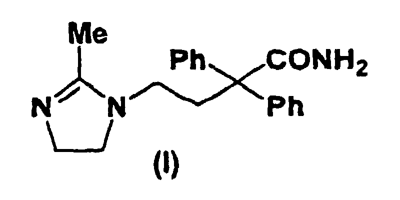 and its pharmacologically acceptable salts, and a novel production intermediately therefor (II).

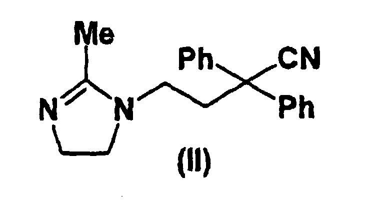

According to the invention, "pharmacologically acceptable salts" include inorganic acid salts with hydrochloric acid, nitric acid, sulfuric acid and the like, organic acid salts with acetic acid, citric acid, fumaric acid, tartaric acid and the like, sulfonic acid salts with methanesulfonic acid, p-toluenesulfonic acid (hereunder referred to as tosylic acid) and the like, and amino acid salts with alanine, leucine, glutamic acid and the like.

Compound (I) and its pharmacologically acceptable salts are sometimes isolated as hydrates, solvates or crystalline polymorphic substances, and these are also encompassed by the present invention.

Since compound (I) has high selectivity for muscarinic M₃ receptor subtypes over muscarinic M₂ receptor subtypes, and has particularly powerful antagonistic action on airway muscarinic M₃ receptor subtypes, it can therefore be used as a preventive or therapeutic agent for COPD and asthma.

Compound (I) can be produced from compound (II) by the methods described in the examples, and pharmacologically acceptable salts of compound (I) can be produced by treatment with inorganic acids such as hydrochloric acid, nitric acid and sulfuric acid, organic acids such as acetic acid, citric acid, fumaric acid and tartaric acid, sulfonic acids such as methanesulfonic acid and tosylic acid, and amino acid salts of alanine, leucine and glutamic acid.

As forms for administration of compound (I) and its pharmacologically acceptable salts there may be mentioned oral forms such as tablets, capsules, granules, powders and syrups, oral and intranasal forms such as inhalants, and parenteral forms such as injections and suppositories.

### Best Mode for Carrying Out the Invention

The present invention will now be explained in further detail by way of the following examples and reference examples which, however, are in no way intended to restrict the invention.

### (Example 1)

### 4-(4,5-Dihydro-2-methyl-1H-imidazolyl)-2,2-diphenylbutyronitrile

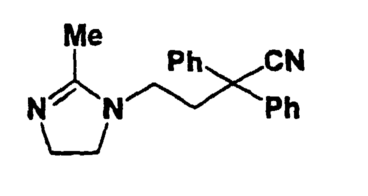

A mixture of 4-bromo-2, 2-diphenylbutyronitrile (2.26 g, 7.53 mmol), 2-methylimidazoline (3.80 g, 45.2 mmol) and acetonitrile (45 ml) was stirred for 4 hours under acetonitrile reflux. The solvent was distilled off under reduced pressure, and the residue was extracted with chloroform, washed with water for 4 times and then dried over anhydrous potassium carbonate. The solvent was distilled off under reduced pressure, and the residue was purified by silica gel column chromatography (eluant: chloroform/methanol = 10/1, followed by 0.5% triethylamine-containing chloroform/methanol = 6/1), to give 1.14 g (49% yield) of the title compound as a yellow oil.
¹H-NMR(CDCl₃) δ ppm:1.76(3H,s), 2.58-2.64(2H,m), 3.17-3.23(2H ,m), 3.28(2H,t,J=9.77Hz), 3.63(2H,t,J=9.77Hz), 7.30-7.40(10H,m).
MS m/z: 304(C₂₀H₂₁N₃+H).

### (Example 2)

### 4-(4,5-Dihydro-2-methyl-1H-imidazolyl)-2,2-diphenylbutylamide

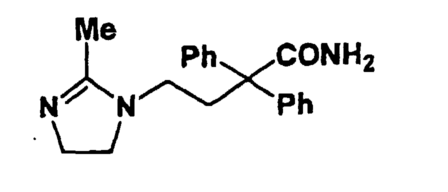

After adding concentrated sulfuric acid (0.7 ml) to a mixture of 4-(4,5-dihydro-2-methyl-1H-imidazolyl)-2,2-diphenylbutyronitrile (0.31 g, 1 mmol) and water (0.3 ml), it was stirred at 120°C for one hour. After cooling, ice and then water were added, the pH was adjusted to 11 with an aqueous 2N sodium hydroxide, and then extracted with chloroform. After drying over anhydrous potassium carbonate, the solvent was distilled off under reduced pressure. The crystalline residue was recrystallized from ethyl acetate to give 0.15 g(47% yield) of the title compound as colorless crystals. Melting point: 160.0-162.5°C
¹H-NMR(CDCl₃) δ ppm:1.73(3H,s), 2.54-2.60(2H,m), 2.99-3.06(2H,m), 3.25(2H,t,J=9.77Hz), 3.59(2H,t,J=9.77Hz), 7.27-7.40(10H,m).
MS m/z: 322(C₂₀H₂₃N₃O+H).

### (Reference Example 1: Compound (VII))

### 3-(4,5-Dihydro-2-methyl-1H-imidazolyl)-1,1-diphenyl-1-propanol

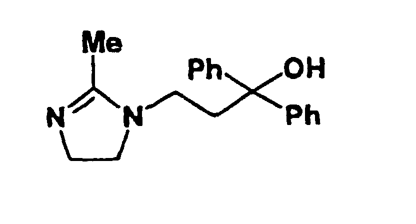

A mixture of 3-chloro-1,1-diphenyl-1-propanol (2.80 g: 11.3 mmol)which synthesized from phenylmagnesium bromide and 3-chloropropiophenone, 2-methylimidazoline (5.70 g, 67.8 mmol) and acetonitrile (30 ml) was stirred for 5 hours under acetonitrile reflux. The solvent was distilled off under reduced pressure, and the residue was extracted twice with chloroform, washed twice with water and then dried over anhydrous potassium carbonate. The solvent was distilled off under reduced pressure and the crystalline residue was collected by suction with ether and dried to give 0.68 g (20% yield) of the title compound as a white crystalline powder.
Melting point: 170.5°C (decomposition)
¹H-NMR(CDCl₃) δ ppm:1.66(3H,s), 2.51(2H,t,J=6.71Hz),3.12(2H,t ,J=6.71Hz),3.28(2H,t,J=9.16Hz),3.59(2H,t,J=9.16Hz),4.50(1H, br),7.21-7.44(10H,m).
MS m/z: 295(C₁₉H₂₂N₂O+H).

### (Reference Example 2: Compound (VIII))

### 2-Cyclohexyl-4-(4,5-dihydro-2-methyl-1H-imidazolyl)-2-phenylbutylamide

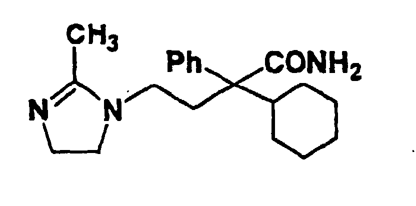

A mixture of 3-cyano-3-cyclohexyl-3-phenyl-1-propyltosylate (0.88 g: 2.21 mmol), 2-methylimidazoline (1.12 g, 13.3 mmol) and acetonitrile (40 ml) was stirred for 7 hours under acetonitrile reflux. After cooling, the solvent was distilled off under reduced pressure, water was added to the residue and then extracted with ethyl acetate. The organic layer was washed with water for three times, rinsed with brine and concentrated under reduced pressure. The residue was purified by silica gel column chromatography (eluant: chloroform/methanol = 10/1 followed by 0.5% triethylamine-containing chloroform/methanol = 6/1) to give 0.40 g (59% yield) of 2-cyclohexyl-4-(4,5-dihydro-2-methyl-1H-imidazolyl)-2-phenylbutyronitrile as a yellow oil.
¹H-NMR(CDCl₃) δ ppm:1.03-1.31(7H,m), 1.66-2.08(5H,m), 1.67(3H,s), 2.33-2.45(1H,m), 2.67-2.77(1H,m), 3.06-3.19(2H,m), 3.24-3.34(1H,m), 3.55-3.63(2H,m), 7.29-7.40(5H,m).

Then, a mixture of the 2-cyclohexyl-4-(4,5-dihydro-2-methyl-1H-imidazolyl)-2-phenylbutyronitrile (0.39 g: 1.26 mmol), 70% sulfuric acid (2 ml) and acetic acid (0.4 ml) was stirred at 120-125°C for one hour. After cooling, ice and then water were added, the pH was adjusted to 12 with a 48% sodium hydroxide aqueous solution, and extracted with chloroform. After drying over anhydrous potassium carbonate, the solvent was distilled off under reduced pressure. The oily residue was crystallized with ethyl acetate and the precipitates were collected by suction with ethyl acetate, then dried to give 0.20g (49% yield) of the title compound as a crystalline powder.
Melting point: 161.5-163.1°C
¹H-NMR(CDCl₃) δ ppm:0.72-1.10(3H,m),1.23-1.41(2H,m),1.6 3-2.17(8H,m), 1.74(3H,s), 2.23-2.34(2H,m), 2.82-3.02(2H,m), 3.21-3.64(2H,m), 5.37(1H,br), 5.65(1H,br), 7.29-7.40(5H,m).
MS m/z: 322(C₂₀H₂₉N₃O+H).

### Effect of the Invention

### 1. Antagonistic action on muscarinic M₃ receptor subtypes in isolated guinea pig trachea

Male Hartley guinea pigs were killed by bleeding after a blow to the head, and the cervical trachea of each was removed while ablating the connective tissue, etc. The esophagus and remaining connective tissue were then amputated and the preparation was prepared by ablation at a spacing of two tracheal cartilage. The preparation was incubated at 37°C, and after suspending it at a resting tension of 1 g in a 5 ml Magnus tank filled with Krebs bicarbonate buffer solution containing indomethacine (1 µM) and aerated with 95% O₂-5% CO₂, it was equilibrated for a period of 60 minutes. The carbachol-induced contraction response was recorded at isotonicity using the cumulative method at a common ratio of 3 from 10⁻⁸ M, with 7 minute intervals between each concentration. The preparation was rapidly washed after recording, and equilibrated for a period of 60 minutes until the next contraction response, and the point at which the EC₅₀ of the carbachol-induced contraction response stabilized was used as a control. The test compound was applied 15 minutes before carbachol application, and the affinity (pA₂) of the test compound was determined according to the Schild method (Arunlakshana, O. and Schild, H.O.: Brit. J. Pharmacol., 1959, 14, 48-58). The results are shown in Table 1.

### 2. Antagonistic action on muscarinic M₃ receptor subtypes in isolated guinea pig bladder

Male Hartley guinea pigs were killed by bleeding after a blow to the head, and after abdominal retraction, the visible protruding part of the bladder in the hypogastric region of each was lightly held with forceps while the bladder trigone was cut for removement and then immersed in a medium. After median dissection, detrusor muscle strips were cut to lengths of 10-15 mm and widths of 3-5 mm. The mucosal tissue was then separated off with ophthalmic scissors for use as a preparation. The preparation was incubated at 37°C, and after suspending it at a resting tension of 1 g in a 10 ml Magnus tank filled with Krebs bicarbonate buffer solution and aerated with 95% O₂-5% CO₂, it was equilibrated for a period of 60 minutes. The carbachol-induced contraction response was recorded at isotonicity using the cumulative method, at a common ratio of 3 from 10⁻⁸ M. The preparation was rapidly washed after recording, and equilibrated for a period of 45 minutes until the next contraction response. The point of stabilization of the EC₅₀ of the carbachol-induced contraction response was used as a control. The test compound was applied 15 minutes before carbachol application. The affinity (pA₂) of the test compound was determined in the same manner as for the trachea. The results are shown in Table 1.

### 3. Antagonistic action on muscarinic M₃ receptor subtypes in isolated guinea pig longitudinal ileum

Male Hartley guinea pigs were killed by bleeding after a blow to the head, and then the ileum of each was rapidly removed while ablating the mesenterium. The contents of the ileum were thoroughly washed, a glass rod with a diameter of 5-7 mm was inserted into the lumen, and the longitudinal muscle alone was cut with a razor along the mesenterium-attached section. A cotton swab was placed at the border between the longitudinal muscle and the circular muscle, and the longitudinal muscle was pulled off as a preparation, in a manner to avoid drying of the tissue. The preparation was incubated at 37°C, and after suspending it at a resting tension of 1 g in a 10 ml organ bath filled with Krebs bicarbonate buffer solution and aerated with 95% O₂-5% CO₂, it was equilibrated for a period of 60 minutes. The carbachol-induced contraction response was recorded at isotonicity using the cumulative method at a common ratio of 3 from 10⁻⁹ M. The preparation was rapidly washed after recording, and equilibrated for a period of 45 minutes until the next contraction response. The point of stabilization of the EC₅₀ of the carbachol-induced contraction response was used as a control. The test compound was applied 15 minutes before carbachol application, and the affinity (pA₂) of the test compound was determined in the same manner as for the trachea. The results are shown in Table 1.

### 4. Antagonistic action on muscarinic M₂ receptor subtypes in isolated guinea pig left atria

Male Hartley guinea pigs were killed by bleeding after a blow to the head, and after rapidly removed the heart and lungs of each, ablation was performed in the order of lungs, connective tissue, etc., heart, and the left and right atria were cut apart to prepare the preparation. The preparation was incubated at 32°C, and then suspended at a resting tension of 0.5 g in a 10 ml organ bath filled with Krebs bicarbonate buffer solution and aerated with 95% O₂-5% CO₂. The contraction upon applying a field electric stimulus (4 Hz, 2 msec, 1.5 x threshold voltage) was then recorded. After standing for a stabilization period of 60 minutes, the carbachol-induced inhibition response was recorded at isometricity using the cumulative method, at a common ratio of 3 from 10⁻⁹ M, with 90 second intervals between each concentration. The preparation was rapidly washed after recording, and equilibrated for a period of 45 minutes until the next inhibition response. The point of stabilization of the EC₅₀ of the carbachol-induced inhibition response used as a control, and the test compound was applied 30 minutes before carbachol application. The affinity (pA₂) of the test compound was determined in the same manner as for the trachea. The results are shown in Table 1.

**Table 1**

| Muscarinic receptors antagonistic action (*in vitro*) | | | | |
|---|---|---|---|---|
| Compound | pA₂ | | | |
| | Trachea M₃ | Bladder M₃ | Ileum M₃ | Left atrium M₂ |
| Compound (I) | 9.7 | 8.9 | 9.4 | 8.4 |
| Compound (IV) | 8.5 | | | 7.3 |
| Compound (VI) | 9 | 8.8 | 9 | 8.3 |
| Compound (VII) | 7.9 | | | 7.2 |
| Compound (VIII) | 8.5 | | | 6.3 |
| Ipratropium | 9 | | 8.7 | 8.4 |
| Atropine | 8.9 | 8.5 | 8.6 | 8.6 |

### 5. Test on guinea pig bronchoconstriction by intravenous administration and inhalation administration

The bronochoconstriction response was measured by the Konzett-Rössler method. (Arch. Exp. Path. Pharmak., 1940, 195, 71-74). Male Hartley guinea pigs (320-440 g) were placed under urethane (1.8 g/kg, i.p.) anesthesia. After the trachea was exposed by cervical median, inserted a tracheal cannula. Animals were ventilated artificially through a tracheal cannula. Spontaneous respiratory movement was stopped by decamethonium (2 mg/kg, i.v.). Bronchoconstriction was measured using a bronchospasm transducer (Ugo Basile Co.). The test compound or control solvent (water, physiological saline or 2% ethanol aqueous solution) was administered through a drug-administering cannula in the left jugular vein, and after 5 minutes, methacholine (3 µg/kg) was administered to induce bronchoconstriction. The effect of the drug on airway resistance was calculated as the contraction percentage (%) based on the total obstruction value (100%) obtained by closure of the tracheal cannula and the dose of the test compound that caused 50% inhibition of bronochoconstriction was recorded as the ID₅₀ value. The results are shown in Table 2.

Administration by inhalation was carried out with the following intravenous administration method using male Hartley guinea pigs (350-504 g). The test compound or physiological saline as a control was administered by inhalation for one minute using an ultrasonic inhalator (OMRON Co.), and after 10 minutes methacholine (3 µg/kg) was administered via a drug-administering intravenous cannula in the left jugular vein to induce bronochoconstriction. The effect of the drug on airway resistance was calculated as the contraction percentage (%) based on the total obstruction value (100%) obtained by closure of the tracheal cannula, and a dose of the test compound that caused 50% inhibition of bronochoconstriction was recorded as the ID₅₀ value. The results are shown in Table 3.

**Table 2**

| Muscarinic receptors antagonistic action (*in vivo*: intravenous administration of test compounds) | | |
|---|---|---|
| Compound | Bronchoconstriction ID₅₀ (µg/kg i.v.) | Saliva secretion ID₅₀ (µg/kg i.v.) |
| Compound (I) | 0.3 | 0.9 |
| Compound (VI) | 1 | 2.6 |
| Ipratropium | 0.9 | |
| Atropine | 3.2 | 6.3 |

**Table 3**

| Muscarinic receptors antagonistic action (*in vivo*: inhalation administration of test compounds) | |
|---|---|
| Compound | Bronchoconstriction ID₅₀ (µg/ml) |
| Compound (I) | 3.3 |
| Ipratropium | 12.3 |

### 6. Test on rat saliva secretion

Male Wistar rats (230-290 g) were anesthetized with urethane (1.2 g/kg, i.p.) and then the right femoral region of each was dissected, and a drug-administering cannula was inserted into the right femoral vein. The test compound or control solvent (water, physiological saline or 2% ethanol aqueous solution) was administered through the cannula, and after 5 minutes, 100 µg/kg of oxotremorine was intravenously administered to induce saliva secretion. Immediately after administration of the oxotremorine, a small cotton ball was inserted into the oral cavity. Salivary secretion was determined for 10 minutes, from the moment of oxotremorine application. The inhibition rate was calculated with respect to the secreted saliva amount in the control group, and a dose of the test compound that produced 50% inhibition of the secreted saliva amount of the control group was determined as the ID₅₀ value. The results are shown in Table 2.

As seen in Table 1, compound (I) exhibited powerful antagonistic action on muscarinic M₃ receptor subtypes and, particularly, airway muscarinic M₃ receptor subtypes, on the order of 10-fold or greater compared to compound (IV).

The muscarinic receptors antagonism was also measured for compound (VII) which is compound (IV) with the cyclohexyl group replaced with phenyl, i.e. compound (I) with the carbamoyl group replaced with hydroxyl, and compound (VIII) which is compound (IV) with the hydroxyl group replaced with carbamoyl, i.e. compound (I) with one of the phenyl groups replaced with cyclohexyl.

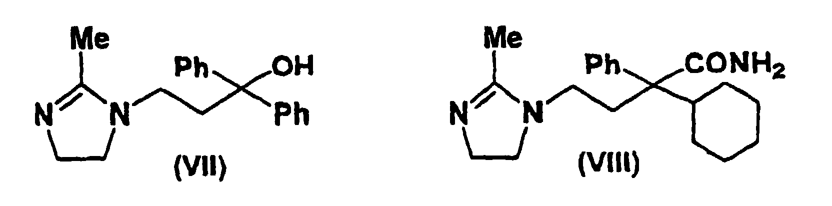

As shown in Table 1, the antagonistic activities of compound (VII) and compound (VIII) on airway muscarinic M₃ receptor subtypes were both weaker than that of compound (I). Compound (VII) was also inferior to compound (I) in terms of receptor selectivity.

Thus, the present inventors have discovered compound (I), which is the conventionally known compound (IV) with the cyclohexyl group and hydroxyl group replaced with phenyl and carbamoyl groups, respectively. Compound (I) has excellent antagonistic activity and receptor selectivity for airway muscarinic M₃ receptor subtypes. It is clear from Table 1 that these excellent results are not easily predictable from compound (IV) and compounds (VII) and (VIII) which have portions thereof replaced. In other words, compound (I) has an effect that cannot be predicted from publicly known compounds or their analogous compounds, and has powerful antagonist action on airway muscarinic M₃ receptor subtypes with excellent receptor selectivity.

Moreover, when the bronochoconstriction ID₅₀ values (relating to the expression of the drug effect) and the saliva secretion ID₅₀ values (relating to the expression of dry mouth as a side-effect of cholinergic antagonists including muscarinic antagonists) in Table 2 are compared, it is seen that the ratio of (saliva secretion ID₅₀ value/tracheal contraction ID₅₀ value) for compound (I) is a larger value than that for atropine or compound (VI). This means that compound (I) produces less of the side effect of dry mouth than atropine and other similar drugs, and that it is therefore a safer pharmaceutical compound.

Also, as shown in Table 3, inhalation of compound (I) exhibited activity of about 3.7 times that of ipratropium, which is currently on the market. This means that compound (I) is a compound that will exhibit an effect that is superior to ipratropium for inhalation in the clinic.

### Industrial Applicability

As explained above, compound (I) exhibits specific antagonism for airway muscarinic M₃ receptor subtypes. In comparison to various control compounds (compound (IV), (VI), (VII), (VIII), ipratropium and atropine), compound (I) has superior receptor and tissue specificity as well as more powerful antagonistic activity on airway muscarinic M₃ receptor subtypes, and is therefore highly useful as a safe and powerful preventive or therapeutic agent for COPD or asthma.

## Claims

1. 4-(4,5-Dihydro-2-methyl-1H-imidazolyl)-2,2-diphenylbutylamide represented by the following chemical formula, 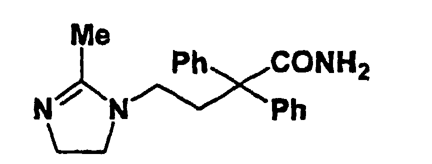 or a pharmacologically acceptable salt thereof.

2. A pharmaceutical composition for prevention or therapy of a disease related to muscarinic M₃ receptor subtypes, which contains 4-(4,5-dihydro-2-methyl-1H-imidazolyl)-2,2-diphenylbutylamide or a pharmacologically acceptable salt thereof as an effective component.

3. A pharmaceutical composition according to Claim 2, wherein the disease related to muscarinic M₃ receptor subtypes is chronic obstructive pulmonary disease or asthma.

4. 4-(4,5-Dihydro-2-methyl-1H-imidazolyl)-2,2-diphenylbutyronitrile represented by the following chemical formula.
